# EUROPEAN PATENT APPLICATION

(11) **EP 4 494 506 A1**
(43) Date of publication of application: **22.01.2025**
(21) Application number: 23769962.4
(22) Date of filing: 09.03.2023
(51) Int. Cl.: A41B 11/00, A61F 13/08, A61B 17/54, A41F 11/00

(54) **SOCK FOR TREATING PLANTAR HEEL PAIN**

(30) Priority: 15.03.2022 ES 202230424 U
(71) Applicant: Fixtoe Device, S.L., 03550 San Juan de Alicante Alicante (ES)
(72) Inventor: LUCAS PICAZO, David, 03550 Alicante (ES); MONZÓ PÉREZ, Francisco, 03550 Alicante (ES)
(74) Representative: Ibarra Garcia, Isabel
(86) International application number: PCT/ES2023/070130
(87) International publication number: WO 2023/175212

(57) **Abstract**

The invention discloses a sock for treating plantar heel pain, formed by a main body made of an elastic and compressive material having a thickness between 4 mm and 12 mm, such that the following are defined in the plantar surface of the sock: an area covering the plantar region of the big toe, on which a first piece is arranged having a Shore A hardness of between 35º and 45º and a thickness of 3 mm; an area covering the second to the fifth metatarsal, on which a second wedge-shaped piece is arranged having a Shore A hardness of between 35º and 45º and a thickness of 2 mm; and an area covering the plantar region of the heel, on which a third piece is arranged having a Shore A hardness of between 20º and 30º and a thickness of between 6 mm and 7 mm.

## Description

### OBJECT OF THE INVENTION

The present invention relates to a sock for treating plantar heel pain (pain in the plantar area of the heel or hindfoot), specifically intended to offer a conservative approach to plantar fasciopathy (plantar fasciosis and/or fasciitis) or other causes responsible for pain in the sole of the foot of a mechanical nature, that is easy for the patient to put on, highly effective, and durable.

### BACKGROUND OF THE INVENTION

As is known, plantar fasciopathy (plantar fasciitis and/or fasciosis) is the foot pathology with the highest incidence rate in the adult population worldwide and is the main cause of pain of a mechanical origin in the plantar heel (plantar heel pain) of the feet.

From an anatomical viewpoint, the plantar fascia is a ligamentous fiber structure that originates in the plantar heel and extends longitudinally towards the plantar base of the five toes where it is inserted. Biomechanically, it performs several functions in the foot, but the most important is to assist in maintaining the stability of the internal and external arch of the foot during loading activities and thus prevent progressive flattening.

In the field of sports podiatry, plantar fasciopathy is the most common foot injury in athletes. It is generally associated with the presence of pronated feet or feet with medially deviated subtalar axes under loading conditions.

The etiology as a study of the causes that can bring about tissue damage in the plantar fascia are various in number, highlighting the following etiological factors, both internal and external:
- Pronated feet and/or feet with medial deviations of the subtalar rotation axis.
- Prolonged standing.
- Shortening of the gastroc-soleus musculature (gastroc-soleus equinus).
- Inflammatory joint diseases such as degenerative and/or inflammatory arthropathies.
- Overweight.
- Excessive use of shoes with high heels and/or excessively flat shoes.

Clinically speaking, the main symptoms of plantar fasciopathy are the following:
- Pain in the plantar and/or medial plantar area of the heel with digitopunction and during loading activities.
- Pain with mechanical characteristics that increases after a prolonged period of rest and improves after the first steps and worsens at the end of the day.
- Pain and morning stiffness.
- Pain that increases during prolonged standing.
- It improves with rest, taking NSAIDs (non-steroidal anti-inflammatory drugs), cryotherapy, massages, and stretching of the musculature in the sole of the foot and gastroc-soleus musculature, use of functional bandages such as low-dye taping and customized orthopedic insoles, etc.
- It improves with the use of high-heeled shoes or cushioning shoes and worsens with the use of excessively flat shoes.
- It may be accompanied by the patient's sensation of a sprained foot.
- Inability to walk barefoot or on hard surfaces.
- Single heel rise test and positive heel stance test.

The differential diagnosis associated with plantar fasciopathy may be the following:
- Calcaneal stress fracture and/or calcaneal bone stress syndrome. Calcaneal compression syndrome.
- Partial and/or total plantar fascia tear.
- Calcaneal spur.
- Baxter's neuropathy. Nerve entrapment syndromes.
- Tarsal tunnel syndrome.
- Rheumatoid arthritis. Inflammatory arthropathies. Fibromyalgia.
- Osteitis of the medial plantar tubercle of the calcaneus.
- Heel fat pad atrophy.
- Heel Fat Pad Syndrome.
- Tendinopathy and/or myositis of the plantar flexor brevis.
- Myositis of the abductor hallucis.

Currently, when a plantar fasciopathy process (plantar fasciitis and/or fasciosis) is diagnosed, conservative treatment measures are applied as the first therapeutic choice in order to, on the one hand, reduce the painful inflammatory phase and control the biomechanical factors responsible for excess tensile and compressive stress on the plantar fascia, and, on the other hand, to promote tissue remodeling with regenerative therapy.

Among orthopedic conservative treatment alternatives in the short term, low-dye taping in the short term and customized orthopedic insoles associated with appropriate footwear in the medium and long term, are known.

There is ample scientific evidence on the positive effect of low-dye taping as a first choice treatment (4-6 weeks of initial treatment) on the control or reduction of pain mentioned by the patient with plantar heel pain, proving to be one of the most successful and effective treatment alternatives in the short term, on an individual basis, and/or as an adjunct to treatment with a custom orthopedic insole.

The orthopedic insole to be used in conjunction with low-dye taping has different elements to control the tensile stress of the plantar fascia, such as a support means for the internal arch of the foot, a piece of medium-density EVA rubber in the plantar forefoot that covers the subcapital area from the second to the fifth metatarsal, and a medium-density subphalangeal EVA rubber insert or wedge of in the entire big toe or plantar hallux such as CLUFFY^{®} (where the term CLUFFY^{®} refers to a brand name for orthotic wedges, so in the context of the present invention it is used as a noun synonymous with "orthotic wedge") or another KINETIC WEDGE. On the other hand, the compressive stress on the plantar fascia is controlled by means of a damping material such as a SHOCK ABSORBER in the plantar area of the heel.

Although the treatment described in which an orthopedic insole is combined with bandages provides a notable symptomatic improvement in the patient, it has certain drawbacks for the patient, such as discomfort in daily hygiene, the need for specialized personnel to change the bandage every 2-3 days, damage to the skin such as contact dermatitis, etc. All these drawbacks diminish the patient's quality of life.

In addition, treatments with adhesive bandages prevent skin transpiration and are very uncomfortable.

Based on the above, the applicant of the present utility model considers it necessary to provide a device in the form of a sock for the treatment of plantar heel pain that satisfactorily solves the above problems, by providing a conservative therapeutic element with the specific effect required by the different areas of the plantar fascia, and which can be easily applied and removed by patients themselves.

### DESCRIPTION OF THE INVENTION

The sock for treating plantar heel pain or fasciopathy that is proposed, fully and successfully overcomes the aforementioned problems based on a simple and effective solution.

In that sense, the sock that is proposed is intended to provide a treatment that is an alternative to low-dye taping and to solve plantar heel pain originating in the plantar fasciopathy or other causes that generate plantar heel pain of a mechanical nature.

Therefore, according to the essence of the invention, the sock for treating plantar heel pain is made up of a main body made of an elastic and compressive material, having a thickness between 4 and 12 mm, and, as is common in garments of this type, the main body has a toe portion for accommodating the toes. Optionally, the toe portion is divided into at least two compartments, such that a first compartment has a shape matching that of the big toe to accommodate same, and the second compartment allows the rest of the toes to be accommodated together.

Preferably, the elastic and compressive material making up the main body of the sock is a washable and breathable bamboo, polyester, elastane, and/or nylon material. However, the present invention is not limited to these materials, and the sock can be made using any material with elastic and compressive fibers that favors breathing and is washable.

The following at least three different areas are defined in the plantar surface of the sock for treating plantar heel pain, i.e., the inner surface coinciding with the sole of the user's foot, preferably on the outside of the main body:
- an area covering the plantar region of the big toe, on which a first piece is arranged having a Shore A hardness of between 35º and 45º and an additional thickness of 3 mm with respect to the thickness of the main body;
- an area covering the plantar region from the second to the fifth metatarsal, on which a second wedge-shaped piece is arranged having a Shore A hardness of between 35º and 45º and being defined by a decreasing thickness, such that the second piece has an additional thickness of 2 mm with respect to the thickness of the main body at its outer end which decreases until it is equal at its inner end to the thickness of the main body. This second piece has dimensions such that it leaves the head of the first metatarsal free, and it extends to the sulcus of the toes;
- an area covering the plantar region of the heel, on which a third piece is arranged having a Shore A hardness between 20º and 30º and an additional thickness of between 6 mm and 7 mm with respect to the thickness of the main body to dampen the impact of the ground on the calcaneus and insertion of the fascia in the calcaneal plantar tubercle.

It should be noted that Shore hardness is a scale for measuring the elastic hardness of materials, determined from the elastic reaction of the material when an object is dropped on it, and the A scale is used for tires, rubber bands, etc.

Optionally, the following is defined in the plantar surface of the sock: a different fourth area covering the inner arch region of the foot, said area being provided with a plurality of wavy, longitudinal raised patterns which provide a tension greater than the tension provided by the elastic and compressive material of the rest of the main body. Preferably, the longitudinal raised patterns are made of the same material as the main body, but with a higher degree of tension, by way of a spring, providing more stability for the longitudinal arch of the foot and controlling its flattening.

Advantageously, the sock of the invention provides the patient with stability, support, comfort, and rest throughout the leg and the foot.

Additionally, the sock of the invention provides the following advantages:
- It is easy to put on and wear, with a longer mean life than known treatment alternatives.
- It favors the relief and reduction of heel pain associated with plantar fasciopathy or other causes related to heel pain.
- It promotes venous return, thus improving blood circulation in the lower limbs and the feeling of heaviness in daily activity and sports activity.
- It is a breathable and washable material (unlike bandage systems), thus avoiding skin irritations.

### DESCRIPTION OF THE DRAWINGS

To complement the description that will be made below and for the purpose of helping to understand the features of the invention according to preferred, practical exemplary embodiment thereof, a set of drawings is attached as an integral part of said description in which the following is depicted in an illustrative and non-limiting manner:
Figure 1 shows a bottom view of the sock for treating plantar heel pain according to a preferred embodiment of the invention, in which the foot of the patient inside the sock has been represented with a dotted line.

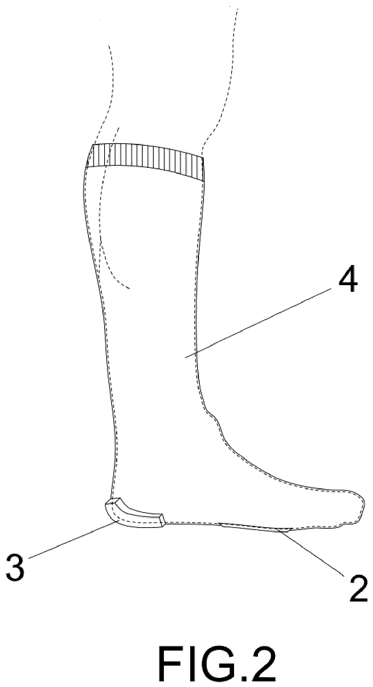

Figure 2 shows a side view of the sock for treating plantar heel pain in the preceding figure placed on the foot of a patient, such that outer side of the foot is shown.
Figure 3 shows a side view of the sock for treating plantar heel pain in the preceding figures placed on the foot of a patient, such that inner side of the foot is shown.
Figure 4 shows a front view of the sock for treating plantar heel pain in the preceding figures placed on the foot of a patient, where the detail of the wedge shape of the second piece can be seen.
Figure 5 shows a perspective view of the sock for treating plantar heel pain in the preceding figures placed on the foot of a patient, where the detail of the toe portion with two compartments can be seen.

### PREFERRED EMBODIMENT OF THE INVENTION

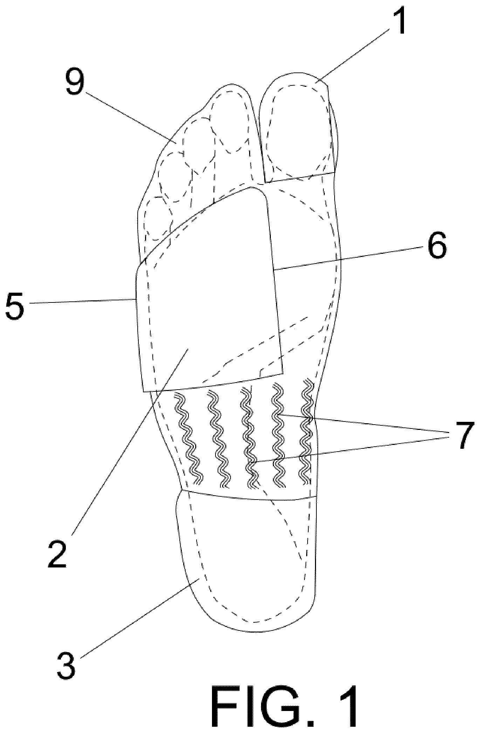

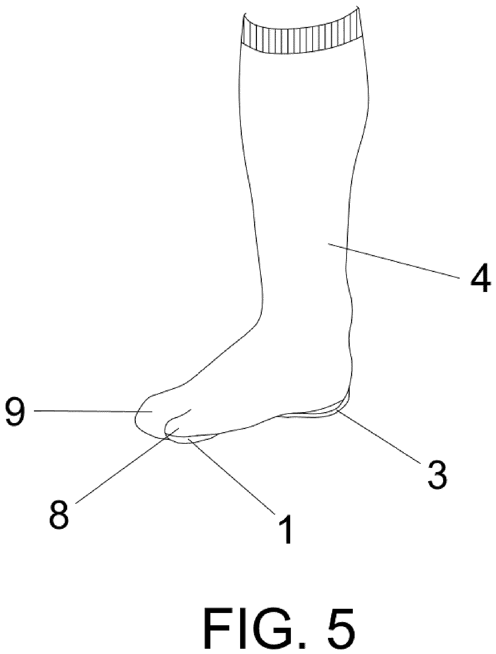

In view of the mentioned figures, it can be seen that, according to a preferred embodiment of the invention, the sock for treating plantar heel pain is based on the conventional structuring of a sock, in which a main body (4) having a toe portion for accommodating the toes is defined. As can be seen in Figures 1, 4, and 5, the toe portion is divided into a first compartment (8) for accommodating the big toe and a second compartment (9) for accommodating the rest of the toes, in order to facilitate proper support of the toes on the different structures or pieces of the sock of the invention.

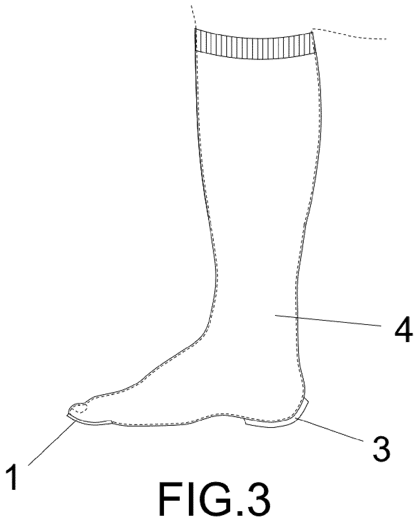

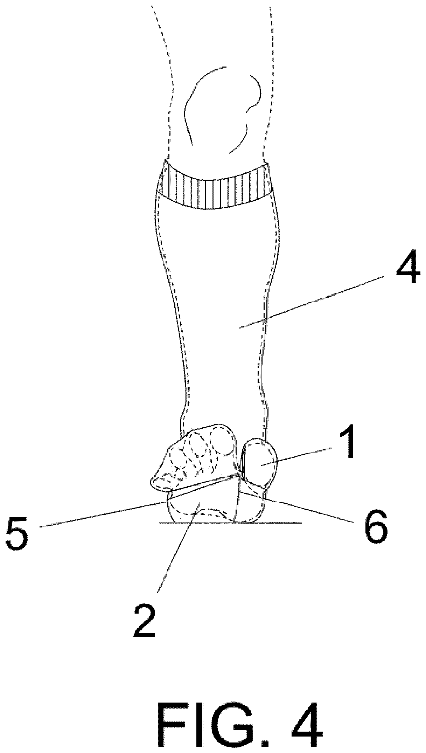

As can be seen in Figure 1, in a preferred embodiment of the invention the following four different areas are defined in the plantar surface of the sock:
- An area covering the plantar region of the big toe, on which a first piece (1) is arranged having a Shore A hardness of between 35º and 45º and an additional thickness of 3 mm with respect to the thickness of the main body (4). This piece can be seen in detail in Figures 1, 3, 4, and 5.
- An area covering the plantar region from the second to the fifth metatarsal, on which a second wedge-shaped piece (2) is arranged having a Shore A hardness of between 35º and 45º and being defined by a decreasing thickness, such that the second piece (2) has an additional thickness of 2 mm with respect to the thickness of the main body (4) at its outer end (5) which decreases until it is equal at its inner end (6) to the thickness of the main body (4). The detail the wedge shape of the second piece (2) can be seen in Figure 4.
- An area covering the plantar region of the heel on which a third piece (3) is arranged having a Shore A hardness of between 20º and 30º and an additional thickness of between 6 mm and 7 mm with respect to the thickness of the main body (4).
- And a fourth area covering the inner arch region of the foot, said area being provided with a plurality of wavy, longitudinal raised patterns (7), wherein the longitudinal raised patterns (7) provide a tension greater than the tension provided by the elastic and compressive material making up the rest of the main body (4), providing improved stability for the arch area of the foot.

The sock for treating plantar heel pain according to the preferred embodiment described provides a therapeutic element with strategically distributed support areas that allows relieving plantar fasciitis with an effective, easy to use device that has no side effects, such as skin irritation.

## Claims

1. A sock for treating plantar heel pain, **characterized in that** it is made up of a main body (4) made of an elastic and compressive material having a thickness between 4 mm and 12 mm, wherein the main body (4) has a toe portion for accommodating the toes, such that the following at least three areas are defined in the plantar surface of the sock:
- an area covering the plantar region of the big toe, on which a first piece (1) is arranged having a Shore A hardness of between 35º and 45º and an additional thickness of 3 mm with respect to the thickness of the main body (4);
- an area covering the plantar region from the second to the fifth metatarsal, on which a second wedge-shaped piece (2) is arranged having a Shore A hardness of between 35º and 45º and being defined by a decreasing thickness, such that the second piece (2) has an additional thickness of 2 mm with respect to the thickness of the main body (4) at its outer end (5) which decreases until it is equal at its inner end (6) to the thickness of the main body (4),
- an area covering the plantar region of the heel, on which a third piece (3) is arranged having a Shore A hardness of between 20º and 30º and an additional thickness of between 6 mm and 7 mm with respect to the thickness of the main body (4).

2. The sock for treating plantar heel pain according to claim 1, **characterized in that** the elastic and compressive material is a bamboo, polyester, elastane, and/or nylon material.

3. The sock for treating plantar heel pain according to claim 1, **characterized in that** the following is defined in the plantar surface of the sock: a fourth area covering the inner arch region of the foot, which is provided with a plurality of wavy, longitudinal raised patterns (7), wherein the longitudinal raised patterns (7) provide a tension greater than the tension provided by the elastic and compressive material making up the rest of the main body (4).

4. The sock for treating plantar heel pain according to claim 3, **characterized in that** the longitudinal raised patterns (7) are made of the same material as the main body (4).

5. The sock for treating plantar heel pain according to any of the preceding claims, **characterized in that** the toe portion is divided into at least two compartments, wherein the first compartment (8) allows the big toe to be accommodated and the second compartment (9) allows the rest of the toes to be accommodated.
